(19) Europäisches Patentamt

European Patent Office

Office européen des brevets

(11) EP 1 724 337 A1

(12) EUROPEAN PATENT APPLICATION

(43) Date of publication:
22.11.2006 Bulletin 2006/47

(51) Int Cl.:
C12N 1/04 (2006.01)    C12N 1/12 (2006.01)
C12P 23/00 (2006.01)

(21) Application number: 06252541.5

(22) Date of filing: 16.05.2006

(84) Designated Contracting States:
AT BE BG CH CY CZ DE DK EE ES FI FR GB GR
HU IE IS IT LI LT LU LV MC NL PL PT RO SE SI
SK TR
Designated Extension States:
AL BA HR MK YU

(30) Priority: 17.05.2005 JP 2005143523

(71) Applicant: YAMAHA HATSUDOKI KABUSHIKI
KAISHA
Iwata-shi, Shizuoka 438-8501 (JP)

(72) Inventor: Ishikura, Masaharu,
c/o Yamaha Hatsudoki K.K.
Iwata-shi,
Shizuoka 438-8501 (JP)

(74) Representative: Schlich, George William et al
HLBBshaw
Shaw House
Pegler Way
Crawley
RH11 7AF (GB)

(54) **Method for preserving xanthophyll in algal cells**

(57)    A method for preserving xanthophyll in algal cells comprises the steps of cultivating algal cells containing xanthophyll and then collecting the algal cells; and drying the collected algal cells so that they have a moisture content of 3 wt% or less. Preferably, drying is performed using spray drying and under drying conditions such that the temperature of air discharged in a spray drier is 125°C or more. In such dry algal cells, xanthophyll can be preserved stably for a long term.

**Description**

1. Field of the Invention

The present invention relates to a method for preserving xanthophyll in algal cells containing xanthophyll, in particular, astaxanthin. Furthermore, the present invention relates to a method for increasing the storage stability of xanthophyll in xanthophyll-containing dry algal cells.

2. Description of the Related Art

Xanthophyll (e.g., astaxanthin, canthaxanthin, zeaxanthin, adonirubin, adonixanthin, cryptoxanthin, and the like), which is a carotenoid, has been used for various purposes. For example, astaxanthin, which is a carotenoid imparting a red color, is known to have a potent antioxidative effect. Thus, it is used as a feed for enhancing the color of fish, a pigment in food, a cosmetic, a health food product, and the like. Examples of the feeds containing astaxanthin include a feed obtained by treating *Phaffia* yeast with NaOH (Japanese Laid-Open Patent Publication No. 6-105657) and a feed obtained by freeze-drying broken algal cells and pulverizing the freeze-dried algal cells (Japanese Laid-Open Patent Publication No. 3-83577).

Astaxanthin can be chemically synthesized or extracted from naturally occurring products, for example, from *Eucarida* such as euphausiids and *Pandalus borealis,* from *Phaffia* yeast, and from algae. When astaxanthin is extracted from crustaceans, such as euphausiids, *Pandalus borealis,* and crabs, or from *Phaffia* yeast, the yield is very low because of their low astaxanthin content. For this reason, astaxanthin is generally extracted from algae, e.g., green algae belonging to the genus *Haematococcus,* having a high astaxanthin content.

Astaxanthin is unstable to heat, oxygen, light, and the like, and a culture suspension of algal cells cannot be stored as it is. Thus, extraction of astaxanthin is often performed immediately after cultivation of algal cells (e.g., see Japanese Laid-Open Patent Publication Nos. 9-111139 and 53-38653). In this case, it is necessary that a culture apparatus and an extraction apparatus be located at the same place. If large quantities of algal cells are cultivated at one time, then it becomes necessary to temporarily store a culture suspension or an extract. It is required to store the culture suspension or the extract at a low temperature under a nitrogen atmosphere with shielding from light so as to prevent oxidation and decomposition of astaxanthin. Moreover, it also becomes necessary to prevent putrefaction, for example, due to bacterial growth. Thus, additional storage equipment is needed, which results in an increase in the cost of the extraction step.

In order to solve the foregoing problem, there is a known method of drying algal cells containing astaxanthin. Japanese Laid-Open Patent Publication No. 7-8212 describes a method of drying algal cells containing a carotenoid and breaking up the dry algal cells in an edible vegetable oil. Also, Japanese Laid-Open Patent Publication No. 2004-129504 describes a process of drying algal cells containing astaxanthin. However, Japanese Laid-Open Patent Publication Nos. 7-8212 and 2004-129504 mentioned above do not discuss the stability of astaxanthin itself in the presence of heat, oxygen, light, and the like during drying. For example, according to a study by the inventor of the present invention, it was found that when drying was simply performed using the drying method described in Japanese Laid-Open Patent Publication No. 2004-129504, astaxanthin in the algal cells was decomposed and thus the astaxanthin content was decreased.

Furthermore, a method for improving long-term storage stability of astaxanthin by mixing broken algal cells with various types of additives to form a powdery composition is known (WO 2002/077105). However, in this method, additives such as a surfactant and an antioxidant are required, so that the cost is increased. Alternatively, because the surfactant plays a role in helping the additives to contaminate the extract, it is necessary to remove these additives from the extract thereafter. Therefore, the method by using the additives is not preferable.

Thus, a method for preserving xanthophylls in algal cells stably for a long term in a simple manner has not yet been known.

SUMMARY OF THE INVENTION

It is an object of the present invention to provide a method for preserving xanthophyll in xanthophyll-containing algal cells and thereby to provide a method for improving the storage stability of xanthophyll, in particular, astaxanthin, in xanthophyll-containing dry algal cells.

The present invention provides a method for preserving a xanthophyll in an algal cell, the method comprising:

cultivating algal cells containing a xanthophyll and then collecting the algal cells; and
drying the collected algal cells so that the moisture content of the collected algal cells reaches a value of 3 wt% or less.

The present invention also provides a method for increasing the storage stability of a xanthophyll in xanthophyll-containing dry algal cells, the method comprising drying xanthophyll-containing algal cell so that the moisture content in the algal cells reaches a value of 3 wt% or less.

In a particular embodiment, the moisture content of the algal cells reaches a value of 2 wt% or less.

In one embodiment, according to any of these methods, the xanthophyll is astaxanthin.

In a further embodiment, according to any of these methods, the algal cell is derived from a green alga belonging to the genus *Haematococcus.*

In a further embodiment, according to any of these methods, the green alga is *Haematococcus pluvialis.*

In another embodiment, according to any of these methods, the drying is performed by spray drying.

In a further embodiment, according to any of methods, the spray drying is performed using a spray drying apparatus, and the temperature of air discharged in the spray drying apparatus is adjusted to 125°C or more.

The present invention further provides a method for manufacturing a xanthophyll, the method comprising any one of the methods described above.

The present invention further provides a dried algal cell containing xanthophyll, having a moisture content of 3 wt% or less.

In embodiments, dried algal cells of the invention have a moisture content of 2 wt% or less.

The present invention further provides a dried algal cell containing xanthophyll, as desribed above or as obtained by any one of the methods described above.

The present invention further provides a fish food comprising dried algal cells containing xanthophyll, as described above or as obtained by any of the methods above.

The present invention further provides the use of a dried algal cell containing xanthophyll, as described above or as obtained by any of the methods above, in manufacture of a fish food.

According to the method of the present invention, xanthophyll in xanthophyll-containing dry algal cells can be kept stable over a long term at room temperature (i.e., without freezing or cooling). Xanthophyll-containing dry algal cells obtained by the method of the present invention can be preserved for a long term at room temperature, and may be suitably used for extraction with subsequent purification of xanthophyll, in particular, astaxanthin. Thus, for example, the step of cultivating astaxanthin-containing algal cells and the step of extracting and purifying astaxanthin can be separated, and it becomes possible to manufacture astaxanthin efficiently at a low cost. Moreover, according to the method of the present invention, astaxanthin in high concentrations can be preserved conveniently, so that astaxanthin can be extracted and collected efficiently. Furthermore, when astaxanthin-containing dry algal cells obtained by the method of the present invention are used as fish feeds, astaxanthin in the feeds can be preserved stably for a long term, so that an effect of enhancing the color of fish is improved.

BRIEF DESCRIPTION OF THE DRAWINGS

Fig. 1 is a schematic view of a spray drying apparatus that can be used in the method of the present invention.
Fig. 2 presents a graph showing the change in astaxanthin content over time in dry algal cells having various moisture contents.

DETAILED DESCRIPTION OF THE INVENTION

Algal Cells

There is no particular limitation on the algae used in the present invention, as long as the algae can produce xanthophyll. Preferably, green algae, e.g., unicellular algae belonging to the genus *Haematococcus,* are used. Examples of the algae belonging to the genus *Haematococcus* include *Haematococcus pluvialis* (*H. pluvialis*), *Haematococcus lacustris (H lacustris)*, *Haematococcus capensis (H capensis)*, *Haematococcus droebakensi (H. droebakensi)*, and *Haematococcus zimbabwiensis* (*H. zimbabwiensis*).

Examples of *Haematococcus pluvialis* (*H pluvialis*) include the NIES144 strain deposited in the Independent Administrative Institution National Institute for Environmental Studies, the UTEX2505 strain deposited in the Culture Collection of Algae at the University of Texas, U.S.A., and the K0084 strain deposited in the Scandinavian Culture Center for Algae and Protozoa, Botanical Institute, at the University of Copenhagen, Denmark.

Examples of *Haematococcus lacustris* (*H. lacustris*) include the ATCC30402 and ATCC30453 strains deposited in ATCC, the IAM C-392, IAM C-393, IAM C-394, and IAM C-339 strains deposited in the Institute of Applied Microbiology, University of Tokyo, or the UTEX16 and UTEX294 strains.

Examples of *Haematococcus capensis (H. capensis)* include the UTEX LB1023 strain.

Examples of *Haematococcus droebakensi (H. droebakensi)* include the UTEX55 strain.

Examples of *Haematococcus zimbabwiensis (H zimbabwiensis)* include the UTEX LB1758 strain.

Among these, *Haematococcus pluvialis* is preferably used.

Cultivation of Algal Cells Containing Xanthophyll

Algal cells containing xanthophyll can be obtained by cultivating algal cells as described above under such conditions that the algal cells can produce xanthophyll. There is no particular limitation on the conditions for the production of xanthophyll. For example, it is possible to employ a method in which vegetative cells of the algae are grown in a medium containing a nitrogen source and trace metals under bright conditions while bubbling carbon dioxide into the medium, then the cells are subjected to at least one of the following stresses: physical stresses due to light irradiation, biological stresses due to nutrient starvation, and chemical substance stresses due to addition of hydrogen peroxide and/or an iron compound, and thereby encystment of the vegetative cells is induced. It is also possible to cultivate the algal cells by adding acetic acid as a carbon source and under dark conditions for vegetative growth, and then encyst the algal cells (see Japanese Laid-Open Patent Publication Nos. 3-83577 and 2004-129504, for example).

The obtained algal cells are collected, for example, by centrifugation, and washed with water, if necessary. The concentration of the algal cells is then adjusted so that the solid concentration is about 5 to 40%, preferably 10 to 30%, and the resultant algal cells are subjected to a drying step described below in greater detail.

In the present invention, "xanthophyll" is a general term for a group of carotenoids having an oxygen-containing functional group, and does not refer to a particular compound. In the present invention, "xanthophyll" mainly refers to a xanthophyll produced by algae, and typical examples of such a xanthophyll include astaxanthin, canthaxanthin, zeaxanthin, adonirubin, adonixanthin, cryptoxanthin, and their fatty acid esters.

Drying of Algal Cells

Drying of the algal cells containing xanthophyll is performed so that the moisture content in the dry algal cells becomes 3 wt% or less. If the moisture content in the dry algal cells exceeds 3 wt%, then decomposition of xanthophyll in the dry algal cells proceeds during preservation, resulting in poor storage stability of xanthophyll.

There is no particular limitation on the method for drying. For example, drum drying (see WO 2000/057724, for example), hot air drying (see Japanese Laid-Open Patent Publication No. 2002-119252, for example), freeze-drying (see US Patent No. 6163979, for example), and spray drying can be employed. Although low temperature drying such as freeze-drying is preferable in order to prevent thermal decomposition of xanthophyll, there are problems in that it takes much time for drying and the drying equipment is expensive. In the case of drum drying, hot air drying, or spray drying, xanthophyll may be decomposed by heating, so that the heating temperature and the heating time should be watched carefully.

The drying temperature is preferably as low as possible so that xanthophyll (especially xanthophyll present in the form of an ester with a fatty acid) in the algal cells is prevented from being decomposed, and the temperature of the mixture of algal cells is preferably kept below 100°C. In drum drying and hot air drying, when the heating temperature is set to 100°C or more, the temperature of the mixture of algal cells may increase to 100°C or more. In spray drying, since a slurry of the algal cells is sprayed, the sprayed algal cells may not form a lump. Thus, the algal cells are uniformly dried within a short range of time, so that thermal decomposition of xanthophylls in algal cells can be prevented. Therefore, in the present invention, spray drying is preferably employed.

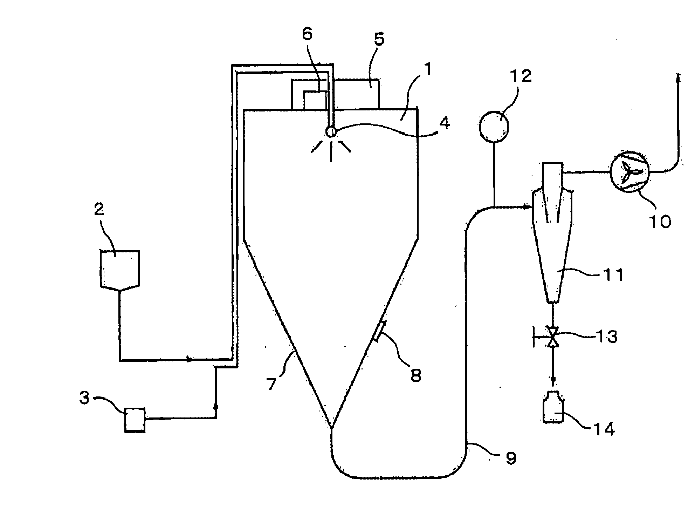

Spray drying can be performed using any spray drying apparatus. Fig. 1 schematically shows a typical spray drying apparatus. The step of drying algal cells using such a spray drying apparatus will be described based on Fig. 1.

A slurry containing algal cells is supplied from a slurry tank (2) to the chamber (1) of a spray drying apparatus. The slurry together with compressed air from a compressor (3) is sprayed into the chamber (1) from the tip of an atomizer (4). Air warmed by a heater (6) within a heater chamber (5) is supplied into the chamber (1), and the sprayed algal cells are dried. The algal cells fall while drying accumulate on a sloped portion (7) in the chamber (1). A hammer (8) provided in the sloped portion (7) gives an impact on the sloped portion (7), and the accumulated algal cells fall into the bottom of the chamber

(1). A fan (10) is provided at the rear end of a channel (9), and when the fan (10) is rotated, the air warmed by the heater (6) is introduced into the chamber (1) from the heater chamber (5) and guides the dry algal cells into a cyclone (collector) (11). A thermometer (12) provided in the channel (9) measures the temperature of the air containing the dry algal cells that are guided from the chamber (1) to the cyclone (collector) (11). In the cyclone (11), the dry algal cells accumulate on the bottom thereof, and the algal cells can be collected into a container (14) by opening a valve (13).

In such a spray drying apparatus, the temperature of the hot air introduced from the heater chamber (6) into the chamber (1) is preferably 200 to 400°C, more preferably 250 to 350°C, even more preferably about 300°C. In the spray drying apparatus, the temperature of the discharged air in the channel (9) is preferably more than 120°C, more preferably 125°C or more, even more preferably 130°C or more. However, if the temperature of the discharged air exceeds 150°C, then the temperature in the mixture of algal cells becomes too high, which may cause thermal decomposition of xanthophyll, so that the temperature of the discharged air is preferably less than 150°C, more preferably 140°C or less. When drying is performed by spray drying as described above, the moisture content in the mixture of dry algal cells becomes 3 wt% or less in a relatively short period of time. In particular, when the xanthophyll is astaxanthin, it is preferable to keep the temperature of the discharged air at less than 150°C. Furthermore, spray drying is preferable because the moisture content in the dry algal cells can be adjusted almost independently of the concentration of the algal cells to be dried, by adjusting the temperature of the discharged air.

The moisture content in the dry algal cells is brought to a value 3 wt% or less by the process described above, more preferably by employing spray drying to dry the algal cells while adjusting the temperature of the discharged air to a temperature of 125°C or more. Even when the obtained dry algal cells are, for example, placed in a glass bottle in air having a humidity of 30 to 40% and stored hermetically at room temperature with shielding from light, decomposition of xanthophyll is minimal. In one embodiment, when algal cells containing astaxanthin and having a moisture content of 3% or less are stored for 50 days at room temperature in air having a humidity of 30 to 40%, the decomposition of astaxanthin is less than 10%. On the other hand, if the moisture content in the dry algal cells exceeds 3%, then the decomposition of astaxanthin reaches as high as 40% when the algal cells are stored for 50 days under the same conditions. As described above, according to the method of the present invention, the storage stability of xanthophyll in algal cells can be increased, and therefore xanthophyll can be preserved for a long term in the algal cells. After drying, in order to prevent further decomposition of xanthophyll, the dry algal cells are preferably stored in a low oxygen atmosphere having a low humidity, e.g., in a vacuum package with aluminum film, so that the dry algal cells do not absorb moisture.

The moisture content in the dry algal cells can be measured by the following method. First, a glass petri dish with a lid is placed in a low temperature drier in which the temperature is adjusted to a predetermined level, and is heated for one hour, and then transferred to a desiccator. The glass petri dish is allowed to cool, and as soon as the temperature of the dish reaches room temperature, the dish with the lid is weighed down to units of 0.1 milligrams. The operation of heating, cooling, and weighing is repeated again to obtain a constant weight ($W_0$, in g). Then, 1 to 2 g of a sample are collected quickly and spread evenly on the glass petri dish, covered with the lid, and weighed accurately ($W_1$, in g). The glass petri dish is then placed in the low temperature drier with the lid open. After the temperature in the low temperature drier reaches 105°C, drying is performed for 3 hours. Thereafter, the glass petri dish is covered quickly with the lid while still inside the drier, transferred to the desiccator, and allowed to cool. As soon as the dish reaches room temperature, the dish, still covered by the lid, is weighed ($W_2$, in g). The moisture content in the sample can be calculated using the following equation:

$$\text{Moisture in sample (\%)} = (W_1 - W_2)/(W_1 - W_0) \times 100$$

Extraction, Collection, and Purification of Xanthophyll

Xanthophyll can be extracted from the dry algal cells having a moisture content of 3 wt% or less and subsequently collected. There is no particular limitation on the method for extracting and collecting xanthophyll, and a method usually used by those skilled in the art can be employed. For example, chemical extraction using an organic solvent such as chloroform, hexane, acetone, methanol, or ethanol, or an edible oil or fat, or physical extraction such as squeezing can be employed. Alternatively, xanthophyll may be extracted and collected using supercritical extraction. After the extraction, xanthophyll can be purified by a method such as concentration and crystallization, or fractionation using a synthetic resin (e.g., styrene-divinylbenzene copolymer).

According to the present invention, in algal cells in which the moisture content is adjusted to 3 wt% or less, xanthophyll can be preserved in high concentrations, so that the amount of astaxanthin that can be extracted and collected from these algal cells does not decrease with storage. Therefore, by utilizing the preservation method or the method for increasing the storage stability of the present invention, it is possible to efficiently collect and manufacture xanthophyll from large quantities of cultivated algae.

Examples

Hereinafter, the present invention will be described in greater detail by means of examples in which algae belonging to the genus *Haematococcus* are used, but it should be appreciated that the present invention is not limited to these examples.

First, a method for measuring xanthophyll, a method for measuring an astaxanthin isomer, and a method for measuring the dry mass of algal cells will be described.

Measurement of Xanthophyll

First, a certain amount of a sample is collected into a microtube designed for exclusive use with a bead beater. After adding zirconia beads to the tube, acetone is added thereto, and the sample is beaten with the bead beater. After the beating step, the sample is separated into a supernatant and a precipitate by centrifugation, and the supernatant (i.e., an acetone fraction) is collected. Acetone is added again to the precipitate, and the same operation as described above is repeated until the color of the precipitate becomes almost completely white. The collected acetone fractions are combined and diluted 100-fold with dimethylsulfoxide (DMSO), and the absorbance at 492 nm ($A_{492}$) and the absorbance at 750 nm ($A_{750}$) are measured. The concentration (in terms of free or unesterified form) of xanthophyll in the collected acetone fractions (sample) can be calculated using the following equation:

$$\text{Xanthophyll concentration in sample (µg/mL)} = 4.5 \times 100 \times (A_{492} - A_{750})$$

Most of the xanthophyll produced by *Haematococcus pluvialis* in the examples below is astaxanthin. The above-described method for measuring xanthophyll is also used for measurement of astaxanthin.

Method for Measuring Astaxanthin Isomer

First, the sample (collected acetone fractions) prepared for the measurement of xanthophyll described above is dissolved in a 0.15 M phosphate buffer (pH 7.0), and cholesterol esterase is added thereto to hydrolyze astaxanthin esters into astaxanthin. The hydrolyzate is analyzed by HPLC with a C18 column. As a detector, an UV detector or a photodiode array detector is used. The HPLC analysis conditions are the same as those described in Kyoko SATO et al., J. Food Hyg. Soc. Japan, Vol. 39, No. 6, pp. 368-374, 1998. Under these conditions, trans-astaxanthin (retention time: about 7.9 minutes), 9-cis-astaxanthin (retention time: about 8.9 minutes), and 13-cis-astaxanthin (retention time: about 9.3 minutes) are eluted in this order. On the commonly accepted assumption that these compounds have the same molar absorption coefficient, the ratio of each compound is obtained by comparing the area values in the chromatogram.

Method for Measuring Concentration of Algal Cells

The concentration of algal cells is measured in the following manner. First, a predetermined amount of the cell suspension is collected and filtered on a GC50 glass fiber filter (made by TOYO·ADVANTEC) by suction. The filter is washed twice with 5 mL of an aqueous solution of hydrochloric acid having a pH of 4 to dissolve inorganic salts. Then, the filter is dried for 3 hours in a thermostatic drier at 105°C and cooled in a vacuum desiccator for one hour to room temperature, and thereafter, the dry weight of the filter is measured. The weight of the GC50 glass fiber filter is preliminarily measured by drying the filter in the thermostatic drier at 105°C for one hour. The concentration of algal cells per unit volume or per unit weight can be calculated by subtracting the dry weight of the filter that was preliminarily measured from the weight of the dried filter to which the algal cells were adsorbed to obtain the dry weight of the algal cells. It should be noted that the concentration of the algal cells in a dispersion of the dry algal cells is measured by the same method as described above, except that washing with an aqueous solution of hydrochloric acid is not performed.

Preparation Example: Culture of Algal Cells Containing Astaxanthin

*Haematococcus pluvialis* K0084 strain (hereinafter, simply referred to as the "K0084 strain") that produces astaxanthin was used. First, 1 L of a MBG-11 medium containing the components shown in Table 1 below was placed in a 1.5 L sealed flat culture flask having a light path of 25 mm, and the K0084 strain was inoculated into the medium so that the initial concentration was 0.6 g/L.

Table 1

| Medium | MBG-11 |
|---|---|
| Components | g/L |
| $NaNO_3$ | 0.41 |
| $K_2HPO_4$ | 0.04 |
| $MgSO_4 \cdot 7H_2O$ | 0.075 |
| $CaCl_2 \cdot 2H_2O$ | 0.036 |
| Citric acid (anhydrous) | 0.006 |
| Ammonium iron (III) citrate | 0.006 |
| $EDTA \cdot 2Na$ | 0.001 |
| $Na_2CO_3$ | 0.02 |
| $CuSO_4 \cdot 5H_2O$ | 0.00286 |
| $H_3BO_4$ | 0.00181 |
| $MnCl_2 \cdot 4H_2O$ | 0.00022 |
| $ZnSO_4 \cdot 7H_2O$ | 0.00008 |
| $Na_2MoO_4$ | 0.000021 |
| $Co(NO_3)_2 \cdot 6H_2O$ | 0.000000494 |

The K0084 strain was cultivated for 5 days under the light irradiation conditions described below while bubbling a gas containing 3 vol% of $CO_2$ at a rate of 600 mL/minute (i.e., at 0.6 vvm) into the medium, adjusting the culture temperature to 25°C and the pH to between 6 and 8.

For irradiation of light, a white fluorescent lamp (made by Matsushita Electric Industrial Co. Ltd., FL40SSW/37) was used as the light source. The intensity of light irradiation was adjusted so that the PPFD in the light receiving direction of the culture tank measured using the LICOR-190SA flat-surface photon sensor was 100 $\mu$mol-p/$m^2$s.

After the cultivation, the K0084 strain changed color from green to brown or blackish brown, and was confirmed to have been encysted.

Then, the same medium (MBG-11 medium) was placed in the same flat culture flask as described above, and the encysted K0084 strain was inoculated so that the initial concentration was 0.6 g/L, and was cultivated for 200 hours under the same culture conditions as described above. By this cultivation, algal cells containing 4.2 wt% of astaxanthin were obtained.

Example: Preparation of Dry Algal Cells

The algal cells obtained in the above-described preparation example were collected by centrifugation, washed with water, adjusted so that the amount of the algal cells was about 18 wt%, and dried by spray drying. By using a spray drier (Anhydro APV PSD52) in which the temperature of the hot air on the inlet side was set to 300°C and the temperature of the discharged air on the outlet side was set to 105°C, 120°C, 130°C, or 140°C, dry algal cells A, B, C, or D were obtained respectively. For each of the dry algal cells A to D, the moisture content, the astaxanthin concentration, and the concentrations of the astaxanthin isomers were measured. The results are shown in Table 2.

Table 2

| Temperature of discharged air (°C) | Dry algal cell | Dry algal cell | | Astaxanthin | | |
|---|---|---|---|---|---|---|
| | | Moisture Moisture content (%) | Astaxanthin (%) | trans(%) | 9-cis(%) | 13-cis(%) |
| 105 | Dry algal cell A | 4.1 | 4.30 | 83.0 | 7.7 | 9.3 |
| 120 | Dry algal cell B | 3.3 | 4.29 | 79.3 | 11.1 | 9.6 |
| 130 | Dry algal cell C | 2.7 | 4.27 | 78.5 | 11.1 | 10.4 |

(continued)

| Temperature of discharged air (°C) | Dry algal cell | Dry algal cell | | Astaxanthin | | |
|---|---|---|---|---|---|---|
| | | Moisture Moisture content (%) | Astaxanthin (%) | trans(%) | 9-cis(%) | 13-cis(%) |
| 140 | Dry algal cell D | 1.9 | 4.18 | 77.8 | 12.0 | 10.2 |

As can be seen from this table, the moisture content in the dry algal cells decreased as the temperature of the discharged air increased, and the moisture content was 1.9% when the temperature of the discharged air was 140°C. It is also seen that in almost all of astaxanthin in the algal cells, both of the double bonds at the 9- and 13-positions are inherently in the trans form. However, as the temperature of the discharged air increased, the 9-cis form and the 13-cis form, which are isomers, tended to increase. This indicates that isomerization of astaxanthin occurs because of heat.

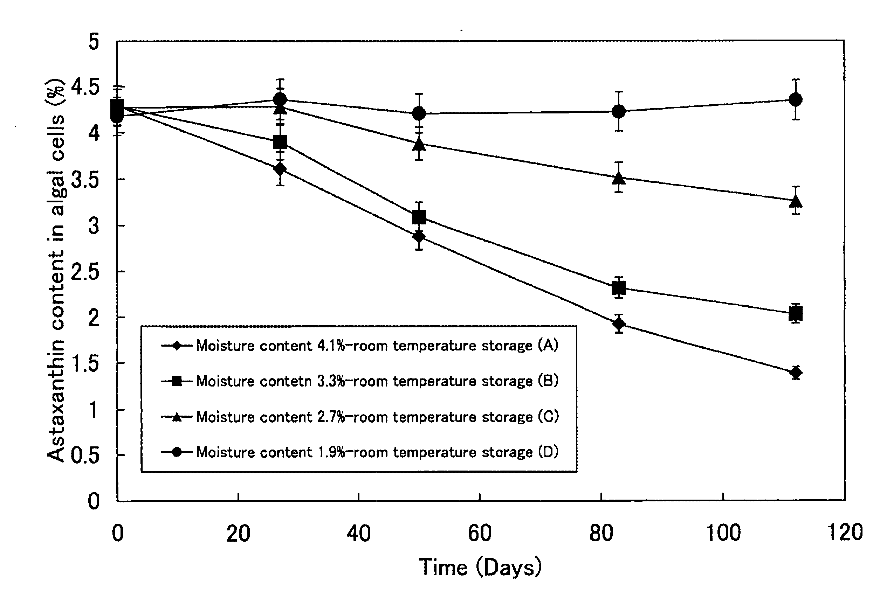

Next, the obtained dry algal cells A to D were placed in glass bottles so that they were present in an amount of 30 vol%. The glass bottles were sealed and stored under conditions of a humidity of 30 to 40% at room temperature, and the respective astaxanthin concentrations were measured over time and taken as indicators of the storage stability. The results are shown in Fig. 2.

As can be seen from Fig. 2, in the dry algal cells C and D having a moisture content of 3 wt% or less, decomposition of astaxanthin was very slow in spite of the fact that those dry algal cells were stored in the glass bottles at room temperature in the ambient atmosphere. In particular, in the dry algal cells D having a moisture content of 2wt% or less, decomposition of astaxanthin hardly occurred even after 80 days or more. In the dry algal cells C, the decomposition rate was 10% or less even on day 50. On the other hand, in both of the dry algal cells A and B having a moisture content of more than 3 wt%, decomposition of astaxanthin occurred, and 30 to 40% of astaxanthin had been decomposed on day 50. In view of these results, it was clear that residual moisture in the dry algal cells has a greater influence on decomposition of astaxanthin than heat during drying of the algal cells. These findings showed that astaxanthin can be stored stably by controlling the moisture content in dry algal cells to be 3 wt% or less. Moreover, when comparing the dry algal cells C and D obtained according to the method of the present invention with the dry algal cells A and B which are comparative examples, there was a large difference in the amount of remaining astaxanthin on and after day 20 from the start of storage. Therefore, the method of the present invention is particularly useful for a storage for 20 days or more.

From the foregoing, it was established that when spray drying is employed, it is most beneficial to set the moisture content of the dried algal cells to 3 wt% or less, which gives overall greater storage stability even when making allowance for some thermal decomposition of astaxanthin.

According to the method of the present invention, xanthophyll (astaxanthin) in dry algal cells containing xanthophyll, in particular, astaxanthin that tends to be decomposed by heat, oxygen, light, and the like, can be stored stably for a long term at room temperature in the air. Thus, the method of the present invention is useful in stabilizing and manufacturing xanthophyll, in particular, astaxanthin. Moreover, according to the present invention, when astaxanthin-containing dry algal cells are used as fish feeds, the effect of enhancing the color of fishes is improved because astaxanthin in the feeds is preserved stably for a long term.

The invention may be embodied in other forms without departing from the spirit or essential characteristics thereof. The embodiments disclosed in this application are to be considered in all respects as illustrative and not limiting. The scope of the invention is indicated by the appended claims rather than by the foregoing description, and all changes which come within the meaning and range of equivalency of the claims are intended to be embraced therein.

**Claims**

1.   A method for preserving a xanthophyll in an algal cell, comprising:

cultivating algal cells containing a xanthophyll and then collecting the algal cells; and
drying the collected algal cells so that they have a moisture content of about 3 wt% or less.

2.   The method of claim 1, wherein the xanthophyll is astaxanthin.

3.   The method of claim 1 or 2, wherein the algal cell is derived from a green alga belonging to the genus *Haematococcus*.

4.   The method of claim 3, wherein the green alga is *Haematococcus pluvialis*.

**5.** The method of any one of claims 1 to 4, wherein the drying is performed by spray drying.

**6.** The method of claim 5, wherein the spray drying is performed using a spray drying apparatus, and the temperature of air discharged in the spray drying apparatus is adjusted to about 125°C or more.

**7.** A method for increasing the preservation stability of a xanthophyll in a xanthophyll-containing dry algal cell, comprising drying xanthophyll-containing algal cells so that they have a moisture content of about 3 wt% or less.

**8.** The method of claim 7, wherein the xanthophyll is astaxanthin.

**9.** The method of claim 7 or 8, wherein the drying is performed by spray drying.

**10.** The method of claim 9, wherein the spray drying is performed using a spray drying apparatus, and the temperature of air discharged in the spray drying apparatus is adjusted to about 125°C or more.

**11.** The method of any one of claims 7 to 10, wherein the algal cell is a green alga belonging to the genus *Haematococcus.*

**12.** The method of claim 11, wherein the green alga is *Haematococcus pluvialis.*

**13.** A method for manufacturing a xanthophyll, comprising the method of any one of claims 7 to 12.

**14.** A dried algal cell, containing a xanthophyll and having a moisture content of about 3 wt% or less.

**15.** The algal cell of claim 14, wherein the xanthophyll is astaxanthin.

**16.** The algal cell of claim 14 or 15, wherein the algal cell is derived from a green alga belonging to the genus *Haematococcus.*

**17.** The algal cell of claim 16, wherein the green alga is *Haematococcus pluvialis.*

**18.** The cell of any of claims 14-17, having a moisture content of about 2 wt% or less.

**19.** A dried algal cell, obtained by the method of any of claims 1 to 13.

**20.** A fish food comprising the algal cells of any of claims 14 to 19 or obtained by the method of any of claims 1 to 13.

**21.** Use of the algal cells of any of claims 14 to 19 or obtained by the method of any of claims 1 to 13, in manufacture of a fish food.

# EP 1 724 337 A1

**European Patent Office**

## EUROPEAN SEARCH REPORT

**Application Number**

EP 06 25 2541

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/091524 A1 (TANAKA NOBUKAZU ET AL) 13 May 2004 (2004-05-13) * the whole document * | 1-21 | INV. C12N1/04 C12N1/12 C12P23/00 |
| X | WO 03/027267 A (PARRY NUTRACEUTICALS LTD; SWATI SEBASTIAN THOMAS; SWAMINATHAN, KUMARAV) 3 April 2003 (2003-04-03) * abstract * * page 10, line 21 * * claim 6 * | 19-21 | |
| X | WO 97/28274 A (BEN-GURION UNIVERSITY OF THE NEGEV; BOUSSIBA, SAMMY; VONSHAK, AVIGAD;) 7 August 1997 (1997-08-07) * page 5, line 23 - page 6, line 3 * * page 18, line 18 - line 20 * * claims 14,18 * | 19-21 | |
| X | MENDES-PINTO M M ET AL: "Evaluation of different cell disruption processes on encysted cells of Haematococcus pluvialis: Effects on astaxanthin recovery and implications for bio-availability" JOURNAL OF APPLIED PHYCOLOGY, vol. 13, no. 1, February 2001 (2001-02), pages 19-24, XP002395326 ISSN: 0921-8971 * abstract * * page 20, left-hand column, paragraph 1 * * page 20, left-hand column, paragraph 4 * | 19-21 | |
| A | EP 1 138 208 A (NIPPON MITSUBISHI OIL CORPORATION) 4 October 2001 (2001-10-04) * the whole document * | | |
| A | US 2004/253664 A1 (LONG THOMAS VEACH) 16 December 2004 (2004-12-16) * abstract * * paragraphs [0017], [0029] * | | |

**TECHNICAL FIELDS SEARCHED (IPC)**

C12N
C12P

The present search report has been drawn up for all claims

| Place of search | Date of completion of the search | Examiner |
|---|---|---|
| The Hague | 21 August 2006 | Huse, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 25 2541

This annex lists the patent family members relating to the patent documents cited in the above-mentioned European search report.
The members are as contained in the European Patent Office EDP file on
The European Patent Office is in no way liable for these particulars which are merely given for the purpose of information.

21-08-2006

| Patent document cited in search report | | Publication date | Patent family member(s) | | Publication date |
|---|---|---|---|---|---|
| US 2004091524 | A1 | 13-05-2004 | WO | 02077105 A1 | 03-10-2002 |
| WO 03027267 | A | 03-04-2003 | US | 2004077036 A1 | 22-04-2004 |
| WO 9728274 | A | 07-08-1997 | AU | 734157 B2 | 07-06-2001 |
| | | | AU | 1455697 A | 22-08-1997 |
| | | | CA | 2246048 A1 | 07-08-1997 |
| | | | DK | 877817 T3 | 22-07-2002 |
| | | | EP | 0877817 A1 | 18-11-1998 |
| | | | ES | 2178749 T3 | 01-01-2003 |
| | | | IL | 116995 A | 31-08-2000 |
| | | | NO | 983423 A | 01-10-1998 |
| | | | US | 6022701 A | 08-02-2000 |
| | | | ZA | 9700830 A | 30-07-1997 |
| EP 1138208 | A | 04-10-2001 | AU | 781683 B2 | 09-06-2005 |
| | | | AU | 7320800 A | 30-04-2001 |
| | | | CA | 2352080 A1 | 05-04-2001 |
| | | | CN | 1337854 A | 27-02-2002 |
| | | | IL | 143391 A | 31-08-2005 |
| | | | WO | 0122833 A1 | 05-04-2001 |
| | | | JP | 2001095500 A | 10-04-2001 |
| | | | NO | 20012629 A | 11-07-2001 |
| | | | US | 6706278 B1 | 16-03-2004 |
| US 2004253664 | A1 | 16-12-2004 | US | 2002015978 A1 | 07-02-2002 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6105657 A
- JP 3083577 A
- JP 9111139 A
- JP 53038653 A
- JP 7008212 A
- JP 2004129504 A
- WO 2002077105 A
- WO 2000057724 A
- JP 2002119252 A
- US 6163979 A

**Non-patent literature cited in the description**

- **KYOKO SATO et al.** *J. Food Hyg. Soc. Japan,* 1998, vol. 39 (6), 368-374